# EUROPÄISCHE PATENTSCHRIFT

(11) **EP 0 948 356 B1**
(45) Veröffentlichungstag und Bekanntmachung des Hinweises auf die Patenterteilung: **22.03.2006**
(21) Anmeldenummer: 97954419.4
(22) Anmeldetag: 18.12.1997
(51) Int. Cl.: A61K 39/395, G01N 33/574, G01N 33/577, C07K 16/30

(54) **MITTEL ZUR IDENTIFIZIERUNG UND THERAPIE METASTASIERENDER TUMORE**
AGENT FOR IDENTIFYING AND TREATING METASTASIZING TUMOURS
AGENT POUR L'IDENTIFICATION ET LE TRAITEMENT DE TUMEURS PRODUISANT DES METASTASES

(30) Priorität: 18.12.1996 DE 19652815
(43) Veröffentlichungstag der Anmeldung: 13.10.1999
(73) Patentinhaber: Forschungszentrum Karlsruhe GmbH, 76133 Karlsruhe (DE)
(72) Erfinder: SLEEMAN, Jonathan, D-76646 Bruchsal (DE); PONTA, Helmut, D-76356 Weingarten (DE); HERRLICH, Peter, D-76229 Karlsruhe (DE); UNTAE, Kim, Snyder, NY 14226 (US)
(74) Vertreter: Fitzner, Uwe
(86) Internationale Anmeldenummer: PCT/EP1997/007105
(87) Internationale Veröffentlichungsnummer: WO 1998/026801

(56) Entgegenhaltungen:
- EP-A- 0 538 754
- DD-A- 260 515
- K. MYERS ET AL.: "Isolation of a cDNA encoding 5T4 oncofetal trophobast glycoprotein." THE JOURNAL OF BIOLOGICAL CHEMISTRY, Bd. 269, Nr. 12, 25.März 1994, BALTIMORE, MD, USA, Seiten 9319-9324, XP002064468 in der Anmeldung erwähnt
- J. SLEEMAN: "Blutgruppenantigene als Metastasierungsmarker und Auslöser des aktiven Zelltods." NACHRICHTEN FORSCHUNGSZENTRUM KARLSRUHE, Bd. 29, Nr. 3, 1997, KARLSRUHE, DEUTSCHLAND, Seiten 241-243, XP002064469
- DATABASE BIOSIS [Online] BIOSCIENCES INFORMATION SERVICE, PHILADELPHIA, PA, US PREV198172009903, 1981

## Beschreibung

Die vorliegende Erfindung betrifft ein Mittel zur Identifizierung und Unterdrückung des Wachstums von Tumorzellen.

Eine der häufigsten Krankheitsursachen ist in der westlichen Welt das Auftreten von bösartigen Tumoren. Problematisch ist heute nach wie vor die frühzeitige Erkennung der Tumore und deren nachfolgende Behandlung. In den letzten Jahren ist eine Anzahl von Markern zur Feststellung des Tumorwachstums und der Bildung von Metastasen beschrieben worden (vgl. Potter-Jordan, K. und Lippman, M., 1994, Hematol. Oncol. Clin. North Am. 8, 73 bis 100 (1994)). Einige diese Marker, z.B. erbE-2, Cathepsin D., haben sich in einer Vielzahl von Studien als gut brauchbar erwiesen. In jüngster Zeit haben Varianten des Proteins CD44 an Bedeutung gewonnen (Kaufmann et al. Lancet 345, 615 bis 619 (1995), EP 0531300, DE-OS 4134982). Aus EP 351313, CA121: 29448, CA 115: 69417, Medline 95085293, CA 111: 209896 sind schließlich Antigene-Antikörper-Systeme bekannt, bei denen Proteine, die N-glykosidisch gebundene Saccharide enthalten, Verwendung finden. Aufgrund dieser Veröffentlichungen ist bekannt, daß poly- oder monoklonale Antikörper, die spezifisch bestimmte Epitope auf den Tumormarkerproteinen erkennen und damit reagieren und die durch Radioisotope markiert und/oder mit cytocidal oder cytotoxisch wirkenden Stoffen konjugiert werden können, zur Diagnose und Therapie von metastasierenden Tumoren anwendbar sind.

Antikörper reagieren auf spezifische Epitope. In der Regel exprimieren verschiedene Tumoren unterschiedliche Marker, und ein Marker deckt andererseits immer nur ein Subset ab. Für eine Tumorbekämpfung im Rahmen einer Mehrkomponententherapie sind daher mehrere unterschiedliche Marker wünschenswert.

Aufgabe der vorliegenden Erfindung war es demgemäß, ein Mittel zur Identifizierung und zur Unterdrückung des Wachstums von Tumoren zu entwickeln, das möglichst für unterschiedliche Tumore gleichermaßen einsetzbar ist.

Diese Aufgabe wird durch Mittel enthaltend Antikörper, die mit Glycoproteiden menschlicher oder tierischer Herkunft, enthaltend N-glycosidisch gebundene Saccharide des blutgruppenspezifischen Antigen-Typs B2, 3, 4 oder A2 reagieren, zur Verwendung bei der Identifizierung und Unterdrückung des Wachstums von Tumorzellen gelöst.

Für die Zwecke der Erfindung kommt jede beliebige Variante von Proteinen tierischer oder menschlicher Herkunft die mit den spezifischen Sacchariden modifiziert sind, in Betracht.

Unter dem Begriff Antikörper sind mono- oder polyvalente Antikörper und poly- und monoklonale Antikörper zu verstehen, aber auch solche, die Fragmente davon darstellen und Derivate davon, einschließlich der F(ab')2, Fab' und Fab Fragmente, aber auch chimäre Antikörper oder Hybridantikörper mit mindestens zwei Antigen- bzw. Epitopbindungsstellen, oder bispezifische rekombinante Antikörper (beispielsweise Quadrome, Triome), Interspecies-Hybridantikörper, Anti-idiotypische Antikörper und solche davon, die chemisch modifiziert wurden und als Derivate dieser Antikörper zu verstehen sind und die entweder über die bekannten konventionellen Verfahren der Antikörpergewinnung oder über DNA-Rekombination, via Hybridomatechniken oder Antikörper-Engineering oder synthetisch oder semisynthetisch nach an sich bekannter Weise herstellbar sind und Neutralisierung- oder Bindungseigenschaften hinsichtlich der oben beschriebenen und definierten Zuckerketten aufweisen. Aus der vielfältigen Literatur sei nur beispielhaft hingewiesen auf Arbeiten von Köhler, G. & Milstein, C., Nature 256, 495 bis497, 1975; Biocca, S. et al., Embo, J. 9, 101 bis 108, 1990; Bird, R.E. et al., Science 242, 423 bis 426, 1988; Boss, M.A. et al., Nucl. Acids Res. 12, 3791 bis3806, 1984; Boulianne, G.L. et al., Nature 312, 643 bis 646, 1984; Bukovsky, J. & Kennett, R.H., Hybridoma fi, 219 bis228, 1987; Diano, M. et al., Anal. Biochem. 166, 223 bis 229, 1987; Huston, J.S. et al., Proc. Natl. Acad. Sci. USA 85, 5879 bis 5883, 1988; Jones, P.T. et al., Nature 321, 522 bis 525, 1986; Langone, J.J. & Vunakis, H.V. (Hrsg.), Methods Enzymol, 121, Academic Press, London, 1987; Morrison, S. et al., Proc. Natl. Acad. Sci. USA 81, 6851 bis 6855, 1984; Oi, V.T. & Morrison S.L., Bio Techniques 4, 214 bis 221, 1986; Riechmann, L. et al., Nature 332, 323 bis 327, 1988; Tramontano, A. et al., Proc. Natl. Acad. Sci. USA 83, 736 bis 6740, 1986; Wood, C.R. et al., Nature 314, 446 bis 449, 1985.

Ein Verfahren zur Herstellung von agglutinierenden, monoklonalen Antikörpern gegen humane A-Erythrozyten wird beispielsweise in DD 260515 offenbart. Dazu wird ein spezieller Klon SIFIN mAK "Anti-A-1 (21III5/E50)" eingesetzt, der offiziell hinterlegt wurde. Ebenfalls wird in der Arbeit von Voak, D. et al., Vox Sanguinis 39(3), 134-140, 1980 ein kostengünstiges Verfahren zur Herstellung eines monoklonalen IgM-anti-A-Antikörpers beschrieben, der sich zur routinemäßigen Blutgruppenbestimmung am Menschen einsetzen läßt. Allerdings wird in diesen Schriften keine konkrete Verwendung der beschriebenen oder ähnlicher Antikörper zur Identifizierung und Unterdrückung des Wachstums von Tumorzellen offenbart.

Hinsichtlich der Herstellung von polyklonalen Antikörpern gegen Epitope stehen eine Anzahl von Verfahren zur Verfügung. Es können z.B. für diesen Zweck in an sich bekannter Weise verschiedene Tiere durch Injektion mit dem erfindungsgemäßen Glycoproteid, oder Fragmenten davon, immunisiert werden und aus den danach gewonnenen Seren die gewünschten polyklonalen Antikörper nach bekannten Methoden gewonnen und gereinigt werden. Verschiedene Adjuvantien zur Erhöhung der Immunantwort auf die Protein-Gabe können, abhängig von dem für die Immunisierung ausgewählten Tier, ebenfalls verwendet werden - beispielsweise Freund's Adjuvant, Mineralgele wie z.B. Aluminiumhydroxid, oberflächenaktive Substanzen wie z.B. Polyanionen, Peptide, Ölemulsionen, Hemocyanine, Dinitrophenol oder Lysolecithin. Erfindungsgemäß bevorzugt sind monoklonale Antikörper. Besonders bevorzugt ist der Antikörper M - N#1,der aus dem bei der DSMZ, Deutsche Sammlung von Mikroorganismen und Zellkulturen GmbH, Braunschweig hinterlegten Zellkultur DSM ACC 2333 erhältlich ist.

Die für die erfindungsgemäße Verwendung bevorzugten monoklonalen Antikörper können durch jede beliebige Technik erhalten werden, die für die Herstellung von Antikörpern über Kultivierung von Zellinien zur Verfügung stehen. Zu derartigen bekannten Techniken zählen z.B. die von Köhler, G. & Milstein, C., 1975, loc. cit., oder Taggart & Samloff, Science 219, 1228 bis1230, 1983, beschriebenen Verfahren mit Hybridomazellen oder solche mit humanen B Zell Hybridomen (Kozbor et al., Immunology Today 4, 72 bis 79, 1983). Auch die Verwendung von Genbibliotheken aus B-Lymphozyten (Ward et al., 1989; Nature 341, 544-546) und "Kombonatorischen" Bibliotheken auf Phagenvektoren (Mc Cafferty et al., 1990, Nature 348, 552-554; Krang et al.; 1991, PNAS88,4363-4366) zählen zu diesen Verfahren

Die Antikörper können nach bekannten Methoden gereinigt werden, beispielsweise über Immunoabsorptions- oder Immunoaffinitätschromatographie, über HPLC (High Performance Liquid Chromatography) oder Kombinationen davon. Antikörper Fragmente, welche den Idiotyp des Moleküls enthalten, können gleichermaßen nach bekannten Verfahren hergestellt werden. Beispielsweise können F(ab')₂ Fragmente durch Pepsin Verdauung des vollständigen poly- oder monoklonalen Antikörpers erhalten werden. Fab' Fragmente können erhalten werden, indem beispielsweise die Disulfidbrücken des betreffenden F(ab')₂ Fragments reduziert werden und Fab Fragmente können geschaffen werden beispielsweise durch Behandlung der Antikörpermoleküle mit Papain und einem Reduktionsmittel.

Zur Identifizierung und Selektion von Antikörpern, Fragmenten oder Derivaten davon kann jedes bekannte Verfahren verwendet werden. Beispielsweise dadurch, daß diese nach entsprechender Markierung dedektierbar sind, wenn sie an isoliertes oder gereinigtes Antigens gebunden haben oder über Immunprazipitation des z.B. über Polyacrylamidgele gereinigten Antigens, oder dadurch, daß Antikörper gegen die Blutgruppenantigene mit anderen blutgruppenspezifischen Antikörpern um das Binden an Zuckerseitenketten konkurrieren.

Gegenstand der vorliegenden Erfindung ist aber auch die Verwendung von Hybridomzellinien zur Herstellung der Antikörper bzw. einer Präparation für die erfindungsgemäße Verwendung zur Herstellung einer enteral, parenteral, systemisch, lokal und/oder topisch zu verabreichenden Applikationsform, sowie zur Herstellung einer zur Injektion, Infusion oder Perfusion geeigneten Darreichungsform und zur Identifizierung von Tumorzellen in vitro.

Erfindungsgemäß finden Mittel mit derartigen Antikörpern Verwendung bei Tumorerkrankungen bei Tier und Mensch, die die Prävention oder Prophylaxe, Kontrolle, Diagnose oder Behandlung derselben umfassen.

Die erfindungsgemäße Verwendung der Antikörper beruht auf der überraschenden Beobachtung, daß Proteine mit N-glycosidisch gebundenen Sacchariden, die zu den blutgruppenspezifischen Antigenen des Typs B2, 3, 4 oder Typ A2 gehören,die Identifizierung und Behandlung verschiedenartiger Tumore erlaubt. Experimente haben gezeigt, daß nicht beliebige Glycoproteine zur Herstellung der Antikörper eingesetzt werden können. So haben sich Proteine mit O-glycosidisch gebundenen Sacchariden als ungeeignet erwiesen. Somit ist insbesondere der klinische Einsatz der erfindungsgemäßen Antikörper für eine Vielzahl von Störungen, Erkrankungen und Zuständen besonders vorteilhaft.

Abhängig von der Art und Ursache der zu behandelnden Erkrankung oder Störung oder des zu beeinflussenden Zustands in einem tierischen oder menschlichen Körper kann es wünschenswert sein, die Antikörperpräparation systemisch, lokal oder topisch an das oder in das betreffende Gewebe oder Organ zu applizieren. Eine systemische Wirkungsweise ist beispielsweise dann erwünscht, wenn unterschiedliche Organe oder Organsysteme behandlungsbedürftig sind. Demgegenüber wäre eine lokale Wirkung in Betracht zu ziehen, wenn nur die örtliche Manifestationen eines Tumors zu beeinflussen ist.

Die betreffenden Antikörper können über jede dem Fachmann bekannte enterale oder parenterale Applikationsroute verabreicht werden. Für die systemische Applikation bietet sich beispielsweise die intravenöse, intravaskuläre, intramuskuläre, intraarterielle, intraperitoneale, orale oder intrathecale Route an. Eine eher lokale Verabreichung kann beispielsweise subcutan, intracutan, intrakardial, intralobär, intramedullär, intrapulmonal oder in oder an das zu behandelnde Gewebe (Binde-, Knochen-, Muskel-, Nerven-, Epithel- oder Knochengewebe) erfolgen. Abhängig von der zu erzielenden Dauer und Stärke der immunsuppressiven Wirkung können die Antikörperpräparationen einmal oder mehrmals, auch intermittierend, pro Tag über mehrere Tage, Wochen oder Monate und in unterschiedlichen Dosen verabfolgt werden. Zur Herstellung einer für die genannten Applikationen geeignete Antikörperpräparation können die dem Fachmann bekannten injizierbaren, physiologisch verträglichen Lösungen in steriler Form verwendet werden. Zur Herstellung einer gebrauchsfertigen Lösung zur parenteralen Injektion oder Infusion stehen die bekannten wäßrigen isotonischen Lösungen, beispielsweise Saline oder eine entsprechende Plasmaproteinlösung ohne Gammaglobulin, zur Verfügung. Die Präparation kann aber auch in Form eines Lyophilisates bzw. Trockenpräparates vorliegen, welches mit einer der bekannten injizierbaren Lösungen unmittelbar vor dem Gebrauch unter sterilen Bedingungen rekonstituiert werden kann, z.B. als kit-of-parts. Die endgültige Herstellung einer erfindungsgemäß zu verwendenden Antikörperpräparation für die Injektion, Infusion oder Perfusion erfolgt durch Vermischen von nach bekannten Methoden gereinigten Antikörpern gemäß oben angegebener Definitionen mit einem der genannten physiologisch verträglichen Lösungen, welche gegebenenfalls supplementiert sein können mit bekannten Träger- oder Hilfsstoffen (z.B. Serumalbumine, Dextrose, Natriumbisulfit, EDTA).

Die Menge der zu verabreichenden Antikörper hängt ab von der Art und Schwere der zu behandelnden Krankheit oder Störung oder des zu beeinflussenden Zustands und des betreffenden Patienten (Tier oder Mensch). Auszugehen ist jedoch von einer zu verwendenden Dosierung von 0,5 - 2, vorzugsweise 0,7 - 1,5mg/kg Körpergewicht des betreffenden Antikörpers pro Dosiseinheit.

Im folgenden wird die Erfindung unter Bezugnahme auf den folgenden Versuchsbericht näher beschrieben:

### Materialien und Methoden.

Zellinien: Die NM-081 (Goshetal, 1983, InVitro 19, 919-928), MT-450 (Kim 1986, J. Surg. Opa133,151-165) sowie MTLN2, MTNL3, MTLy, MTPa, MTC (Nevietal, 1982, J. Natl. Cancer Inst.68, 507-517; Lichtmer et al., 1987, Invasion Matastaxis 7, 73-82) Zellinien wurden in DME gezogen ergänzt mit 10 % FCS (Fetal Calf Serum). Die Linien RBA und MTAB wurden von der American Type Culture Collection (ATCC, Nummer CRL 1747 und CRL 1666) bezogen.
Subtraktive Immunisierung: Gewebekulturzellen wurden mit PBS/5mM EDTA geerntet und dann dreimal in PBs gewaschen. Für jede Subtraktion wurden 10 weibliche BalbC/B16 F1 Hybridmäuse, denen zuvor Präserum entnommen worden war, mit 2×10⁶ Tumor- oder Gewebekulturenzellen, welche keine gewünschten Antigene tragen, intracutan injiziert (als Tolerogen, gegen welches Antikörper produziert werden, an denen man nicht interessiert ist). Nach 24h und nach 48h wurden jeder Maus 200mg/kg Cyclophosphamid intra peritoneal injiziert. Drei Wochen später wurden den Mäusen entsprechend nochmals das Tolerogen und Cyclophosphamid injiziert. Zehn Tage hierauf wurden Testblutungen genommen. Drei Wochen nach der letzten Injektion von Tolerogen wurden 2x10⁶ Tumor- oder Gewebekulturzellen, für welche Antikörper hergestellt werden sollen, intracutan injiziert (als Immunogen). Das Immunogen wurde nach jeweils 3 Wochen noch zweimal injiziert. Zehn Tage nach der letzten Immunisierung wurden Testblutungen genommen. Die nach den Tolerogen- und Immunogenimmunisierungen genommenen Testblutungen wurden zusammen mit den jeweiligen Präseren verglichen, wobei Zell-ELISA's mit Tolerogen und Immunogen als Targets eingesetzt wurden. Dadurch war es möglich, jene Mäuse zu identifizieren, in denen die Immunantwort auf Tolerogen maximal zerstört und in denen die Immunantwort auf Immunogen maximal verstärkt war. Diese Mäuse wurden für die Herstellung von Hybridomazellen ausgewählt. Vier Wochen nach der letzten Testblutung wurde den Mäusen die Milz entnommen und die Milzzellen mit SP2/0 Myelomzellen fusioniert.

Monoklonale Antikörper: Hybridomazellen wurden nach der Methode von Harlow und Lane, 1988, Antibodies: A Laboratory manual; Cold Spring Habor Laboratory press, hergestellt. Die Hybrodome wurden in Zell-ELISA's auf Produktion von Antikörper getestet, wobei das Immunogen als Target verwendet wurde. Antikörper wurden aus konditioniertem Medium von im ELISA Test positiven Hybridomazellen hergestellt, wobei Protein G-Agarose (Dianova) als Affinitätssäule verwendet wurde. Zell-ELISA: Targetzellen wurden mittels PBS/5 mM EDTA geerntet und in DMEM (Dulbecco's Modified Eagle's Medium)/10% FCS resuspendiert (2x106/ml). 50 µl Antikörper Testlösung wurden jeweils in U-förmiger Vertiefung von Trägerplatten (96 Vertiefungen pro Platte) pipettiert und 50 µl Aliquots von Zellen wurden dann zugegeben. Die Mischung aus Zellen und Antikörpern wurde bei 37°C für 3h inkubiert und dann dreimal mit 200 µl Aliquots von PBS gewaschen. Primär gebundene Antikörper wurden mit Kanninchen-Antimaus Antikörpern, welche an Merretichperoxidase gekoppelt waren, inkubiert und anschließend mit ABTS (2,2' Azino-di-(3-ethyl-benzthiazolin-sulphonate(6) detektiert.

Immunpräzipitation: Zellen wurden durch Inkubation mit (³⁵S)L-Methionin (500mCi/ml) 4 h in methioninfreiem RPMI-Medium, welches mit dialysiertem FCS, supplementiert war, inkubiert. Für eine pulse- chase-Analyse wurden Zellen mit (³⁵S)L-Methionin (500mCi/ml) über 15 Minuten im methioninfreiem RPMI-Medium, ergänzt mit dialysiertem FCS, inkubiert, dann gewaschen und mit methioninhaltigem Medium weiterinkubiert. Für Immunpräzipitationen wurden Zellen in RIPA (RadioImmunPräzipitation) Puffer lysiert und Aliquots wurden mit 5*µ*g Antikörper immunpräzipitiert. Zur Reinigung von Proteinen, die aus Ulex Europaea Lectin binden, wurden die Zellen mit PBS/1% NP40/1mM PMSF lysiert, zentrifugiert und ergänzt mit dialysiertem FCS, inkubiert, dann gewaschen und mit methioninhaltigem Medium weiterinkubiert. Für Immunpräzipitationen wurden Zellen in RIPA (RadioImmunPräzipitation) Puffer lysiert und Aliquots wurden mit 5*µ*g Antikörper immunpräzipitiert. Zur Reinigung von Proteinen, die aus Ulex Europaea Lectin binden, wurden die Zellen mit PBS/1% NP40/1mM PMSF lysiert, zentrifugiert und die Lysate für eine Stunde bei 4°C mit Ulex Europaea Lectinkügelchen inkubiert. Die Kügelchen wurden dann dreimal mit PBS gewaschen und der überschüssige Puffer entfernt. Die Kügelchen wurden in 1% SDS/20mM Phosphat bei pH 7,0 gekocht. Ein 20facher Überschuß an RIPA-Puffer wurde sodann hinzugefügt und der Überstand wurde für die Immunupräzipation nach der Abtrennung von den Ulex Europaea Lectinkügelchen verwendet. In Experimenten, in denen immunpräzipitierte Proteine mit 0,5U N-Gycosidase F, 2,5mU O-Glycosidase oder 5mU Neuraminidase behandelt wurden, wurde der Antigen/Antikörper-Matrixkügelchenkom- plex zunächst in 100mM Phosphatpuffer, pH 7,0, gewaschen. Puffer wurde entfernt und dann wurden die Matrixkügelchen für 5 Minuten in 5*µ*l 1%SDS/20mM Phosphat, pH 7,0, gekocht. Die Enzyme wurden dann in 45*µ*l 20mM Phosphatpuffer (pH 7,0)/1mM CaSO₄ zugegeben. Die Effektivität der Enzymumsetzung wurde bestätigt durch Vergleich mit ähnlichen Verdauungsprozessen an immunopräzipitiertem CD44v4-v7 Protein, welches von jedem der genannten Enzyme verdaut wird. Immunopräzipitierte Proteine wurden mittels SDS (Sodium Dodecyl Sulfate)-PAGE (Polyacrylamid-Gelelektrophorese) abgetrennt. Gele wurden mit PPO (2,5 Diphenyloxazol) behandelt, und röntgensensitivem Filmmaterial ausgesetzt.
Metastasentests: Weiblichen w/Fu Ratten wurden subcutan 5x10⁵ MT-450 Zellen in PBS injiziert. Die Tiere wurden regelmäßig überwacht bis die Größe ihrer Tumore gesetzliche Grenzen erreicht hatte oder die Ratten moribund wurden. Dann wurden die Tiere getötet und Autopsien vorgenommen. In Therapieexperimenten mit Antikörpern wurden Tumorzellen zusammen mit Antikörpern (200*µ*g/Ratte) in Ratten injiziert. Danach erhielten die Tiere zweimal wöchentlich über vier Wochen 200*µ*g Antikörper subcutan an der Stelle der Tumorzelleninjektion. auf Trägerplatten mit 8 vertieften Kammern (Nunc) herangezogen. Zellen aus Zellsuspensionen wurden an silanbeschichteten Trägerplatten fixiert mittels einer Cytospin Zentrifuge. In beiden Fällen wurden die auf den Trägerplatten fixierten Zellen dreimal mit PBS gewaschen, in 4% Paraformaldehyd fixiert und dann für 30 Minuten in PBS/10%FCS (FPBS) inkubiert. Darauf wurde eine Antikörperlösung (in FPBS) zugegeben und die Inkubation für 2h fortgesetzt. Anschließend wurden die Zellen dreimal mit PBS gewaschen und 1h mit Texas Red konjungierten Kanninchen Anti-Maus Ig, verdünnt in FPBS, inkubiert. Nach zwei Waschvorgängen mit PBS wurden die gefärbten Zellen auf den Chamber slides montiert.

### Bindung an synthetische Oligosaccharide

Blutgruppenspezifische Oligosaccharide, die an einer festen Matrix verankert sind (SYNSORB®), wurden von Chembiomed Ltd. (Edmonton, Canada) bezogen. Die chemische Struktur der Oligosaccharide ist in Tabelle 4 dargestellt. Der mAb M-N#1 (0,2 *µ*g in 200 *µ*l PBS/1& BSA) wurde bei Raumtemperatur eine Stunde mit 10 mg beladenen (oder unbeladenen als Kontrolle) SYNSORB®-Kügelchen inkubiert. Nach Inkubation wurden die Überstände gesammelt und auf Reaktivität gegen Speichelmucine der Blutgruppe B wie folgt getestet. Speichel von Individuen der Blutgruppe B oder Blutgruppe 0 wurde unmittelbar nach Sammlung für 15 Minuten im kochenden Wasserbad erhitzt und anschließend 5 Minuten bei 13.000 g zentrifugiert. Der klare Überstand wurde 1:80 mit 0,1 M Karbonatpuffer pH 9 verdünnt. Mit dieser Verdünnung wurden ELISA Platten (NUNC Immunoplates) 1 Stunde bei Raumtemperatur inkubiert. Nach Waschung der Platten mit PBS wurden diese mit PBS, 3 BSA inkubiert. Je, 100 *µ*l der Überstände, nach Absorption von M-N#1 an die SYNSORB®-Kügelchen wurden in Duplikaten auf die beschichteten ELISA-Platten pipettiert. Nach Inkubation über Nacht bei 0°C wurden die Platten mit PBS gewaschen und anschließend mit 100 *µ*l Ziegen gegen Maus IgG, die alkalische Phosphatase gebunden haben (Sigma, St. Louis, MO, USA), für 1 Stunde bei Raumtemperatur inkubiert. Nach Waschung mit PBS wurden gebundene Antikörper mit p-Nitrophenylphosphat (Sigma) nachgewiesen.

### Ergebnisse und Diskussion

Es ist gelungen, durch subtraktive Immunisierung monoklonale Antikörper herzustellen, welche spezifisch mit Epitopen auf metastasierenden Tumoren von Mammakarzinomzellinien von Ratten reagieren. Mäuse wurden zunächst mit der nicht-metastasierenden Ratten-Mammakarzinomzellinie NM-081 (als Tolerogen) immunisiert. Nach einem bzw. zwei Tagen wurden die Mäuse mit Cyclophosphamid behandelt, um die aktivierten Immunzellen abzutöten. Nach drei Wochen wurde die Injektion von Tolerogen und Cyclophosphamid wiederholt. Nach weiteren drei Wochen wurde den Mäusen die metastasierende Ratten-Mammakarzinomzellinie MT-450 (als Immunogen) appliziert. Die Effektivität der Immunisierung und der Cyclophosphamidbehandlung wurde durch Zell-ELISAs mit Seren, die jeweils vor oder nach Behandlung mit Tolerogen, Cyclophosphamid bzw. Immunogen entnommen wurden, getestet, wobei NM-081 Zellen oder MT-450 Zellen als Targets verwendet wurden. Kein Serum zeigte eine Immunreaktion gegenüber dem Tolerogen 10 Tage nach der zweiten Immunisierung und Cyklophosphamidbehandlung, demonstrierend die Effektivität der Cyclophosphamidbehandlung. Alle diese Seren zeigten aber eine verstärkte Reaktivität gegenüber dem Immunogen.

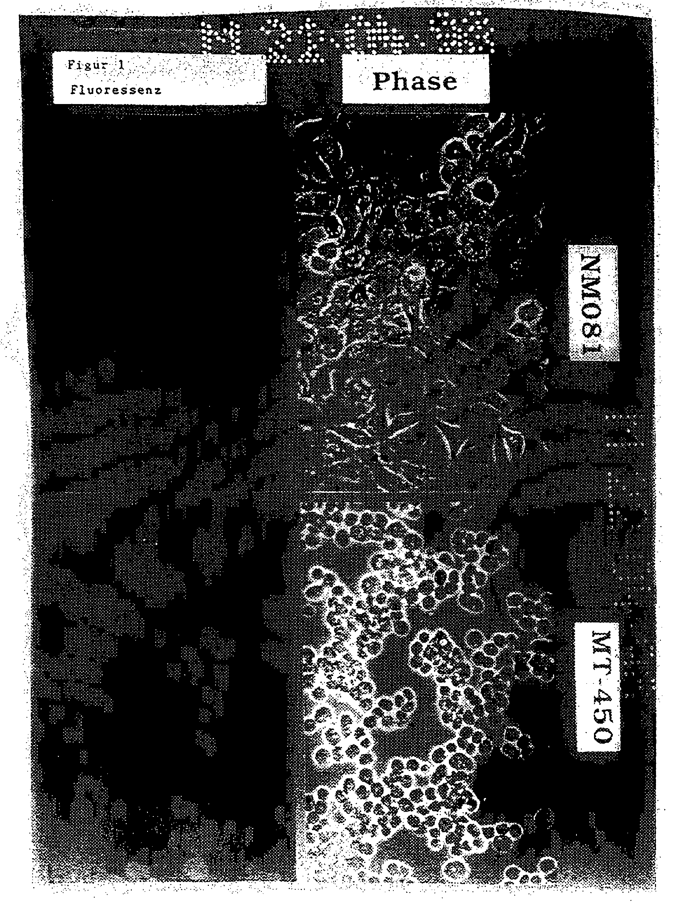

Milzzellen von immunisierten Mäusen wurden zur Generierung von Hybridomen verwendet. Keines der Hybridome produzierte Antikörper, welche das Tolerogen alleine erkannten mit der Wirksamkeit der subtraktiven Immunisierung.
Eines der Hybridome produzierte einen Antikörper (M-N#1), der in Zell ELISAs spezifisch nur mit der metastasierenden MT-450 Zellinie reagierte. Dieser Antikörper zeigt auch Immunfluoreszenzanfärbung mit MT-450 Zellen (Fig. 1) und kann für Immunpräzipation verwendet werden.. Er zeigt allerdings keine Reaktivität in Westernblots.

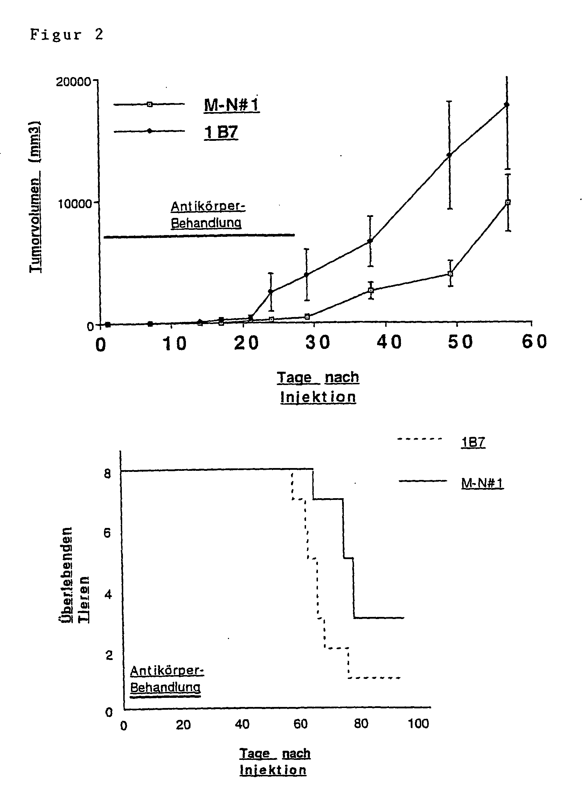

Um zu prüfen, ob der Antikörper nicht nur ein metastasenspezifisch exprimiertes Epitop erkennt, sondern im Stande ist, Tumormetastasierung zu unterbinden, wurden MT-450 Zellen in Brustdrüsengewebe von Ratten injiziert und nachfolgend entweder mit dem M-N#1-Antikörper oder mit einem Kontrollantikörper gleichen Isotyps behandelt. Der M-N#1Antikörper unterdrückte das Wachstum der Tumore, was zum einen dazu führte, daß sich in weniger Tieren als in der Kontrolle Tumore ausbildeten, zum anderen die angewachsenen Tumore deutlich kleiner als in den Kontrolltieren waren. Weiterhin hatte der Antikörper auch hemmende Wirkung auf Metastasenwachstum in den Lymphknoten der tumortragenden Tiere. Auch die Überlebensperiode der mit dem M-N#1-Antikörper behandelten Tier war deutlich länger als für Kontrolltiere (Fig. 2).

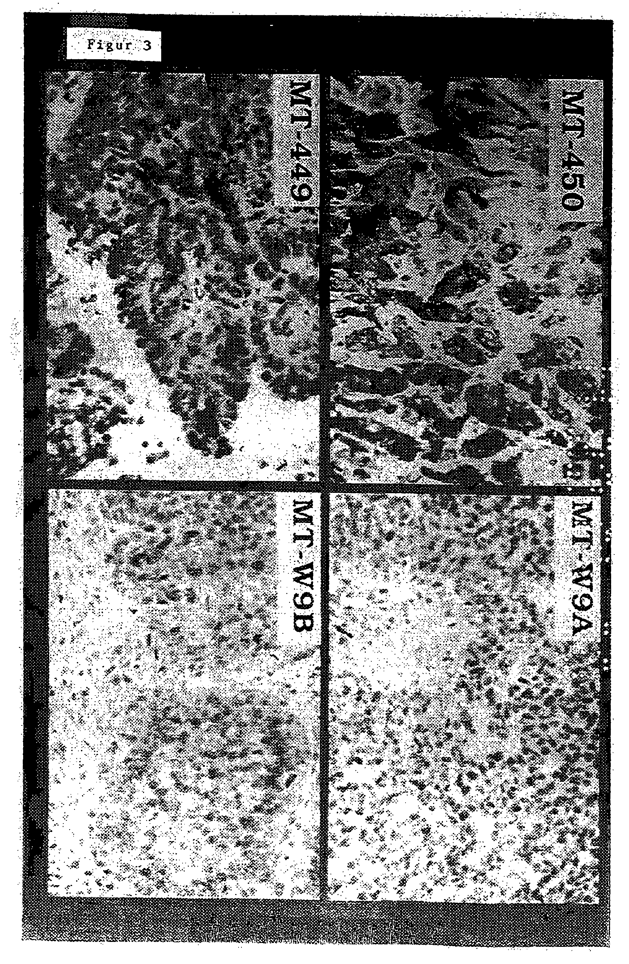

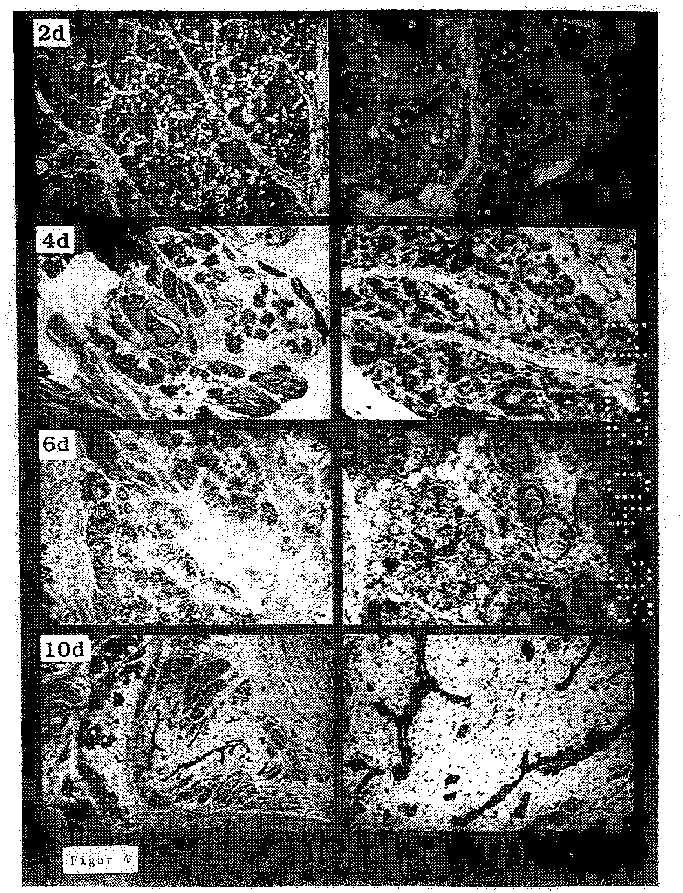

Zur Prüfung der Spezifität des M-N#1-Antikörpers wurden verschiedene Mammatumorzellinien in Gewebekultur und Tumore der MT-W9 Mammatumorserie, von der es Linien mit unterschiedlichem Metastasierungspotential gibt, in Gewwebeschnitten immunhistologisch mit M-N#1-Antikörper angefärbt (Fig. 3). Von den Tumoren der MT-W9 Serie reagierten die Antikörper mit den metastasierenden MT-450 und MT-449, nicht aber mit den nicht-metastasierenden MT-W9A und MT-W9B. Von den Zellen in Kultur reagierten keine der Linien mit geringem Metastasierungspotential (Tabelle 1). Von den metastasierenden Mammakaninomzellinien reagierten einige mit dem Antikörper, einige allerdings nicht. Dies bedeutet, daß die Expression des M-N#1-Epitopes nicht für alle Tumore obligatorisch für den Metastasierungsprozeß ist.
Um zu ermitteln, ob das M-N#1-Antigen auch unter physiologischen Bedingungen in Tieren exprimiert wird, wurden eine Reihe von Rattengeweben auf Reaktivität mit dem M-N#1-Antikörper untersucht. Positiv reagierten Epithelien mit Drüseneigenschaften (Tabelle 2). Leukozyten aus der Milz, dem Thymus oder von Lymphknoten Makrophagen aus dem Peritoneum waren negativ. In Paraffinschnitten wurden das Epithel der Korpus- und Antrumregion des Magens, die azinalen Zellen des Pankreas und die basalen Zellen der Darmkrypten angefärbt. Auch im Knochenmark fand man starke Reaktivität. Eine Ursache, warum das M-N#1-Epitop auf metastasierenden Brustdrüsentumoren exprimiert ist, könnte sein, daß auch unter normalen physiologischen Bedingungen dieses Antigen auf Brustdrüsengewebe vorkommt. Normales Brustdrüsengewebe zeigt keine Reaktion mit dem M-N#1-Antikörper, auch nicht während Schwangerschaft und Laktation. Interessanterweise findet man starke Expression des M-N#1Epitops zwei Tage nach Ende der Laktation, zu Beginn der Rückbildung der Mamma und während der Rückbildungsphase (Fig. 4).

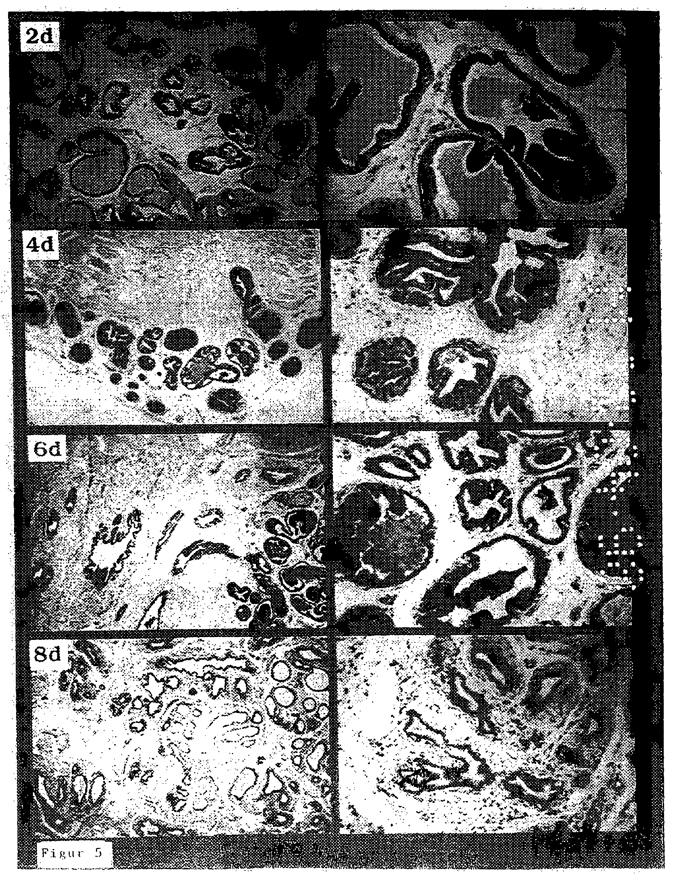

Ein Charakteristikum dieser Rückbildungsphase ist die Entfernung von Zellen durch Apoptose (programmierter Zelltod). Auch andere Organe können sich durch Apoptose rückbilden. Dazu gehört die Vorstehdrüse nach Kastration. Auch das Gewebe der Vorstehdrüse zeigt, wie normales Brustdrüsengewebe, keine Expression des M-N#1-Epitops. Bereits zwei Tage nach Kastration, mit Beginn der Rückbildung der Drüse, findet man starke Expression von M-N#1-Antigen und diese Expression wird während der Regressionsperiode aufrechterhalten (Fig. 5). Diese Daten suggerieren, daß das M-N#1-Antigen auf apoptotischem Gewebe exprimiert wird.

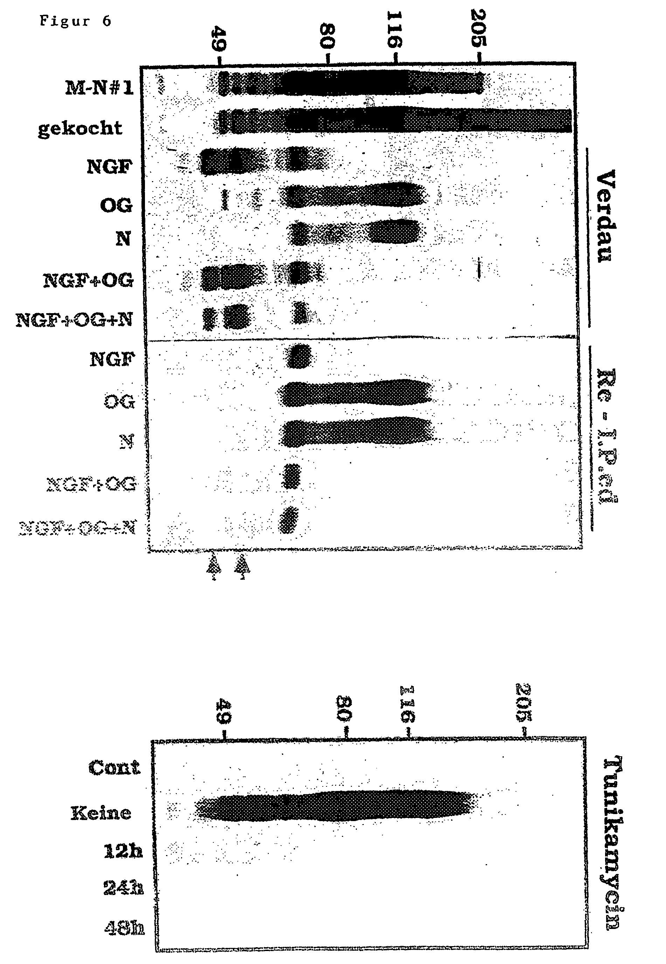

Zur Identifizierung des Antigens, welches mit dem M-N#1-Antikörper interagiert, wurde reaktives Material aus einem Zellysat mit M-N#1 Antikörper immunpräzipitiert und anschließend das präzipitierte Material gelelektrophoretisch aufgetrennt. Das präzipierte Material trennte sich nicht in diskrete Banden auf, sondern war als "Schmier" sichtbar, suggerierend, daß es sich um post-translational modifiziertes Material handeln könnte. Das immunpräzipitierte Material wurde daher der Behandlung mit verschiedenen Glykosidasen unterzogen. Nur die Behandlung mit N-GlykosidaseF hatte Effekt und führte zu einer Reduktion des "Schmiers" auf zwei Banden mit einem scheinbaren Molekulargewicht von 49/51 kD (Fig. 6). Chondroitinsulfat und Heparansulfat-Modifikationen wurden ausgeschlossen.

Interessanterweise konnte M-N#1-Antigen nach Behandlung mit N-GlykosidaseF Behandlung nicht mehr mit M-N#1-Antikörpern immunpräzipitiert werden. Da nach Hitzebehandlung des MT-450 Lysates die Immunpräzipitierbarkeit des M-N#1-Antigens nicht zerstörte wurde, ist das von M-N#1 erkannte Antigen wahrscheinlich ein Zuckerrest. Dies wurde bestätigt, indem auch Behandlung der MT-450 Zellen mit Tunikamycin zu Verlust des immunpräzipitierbaren Materials führt (Fig. 6).

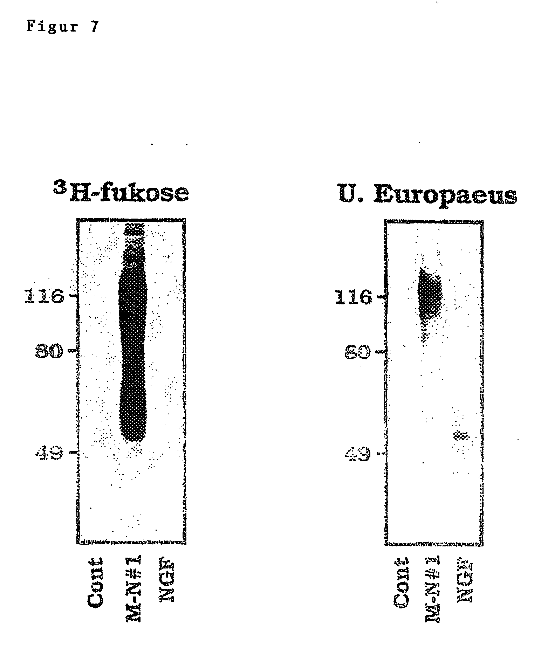

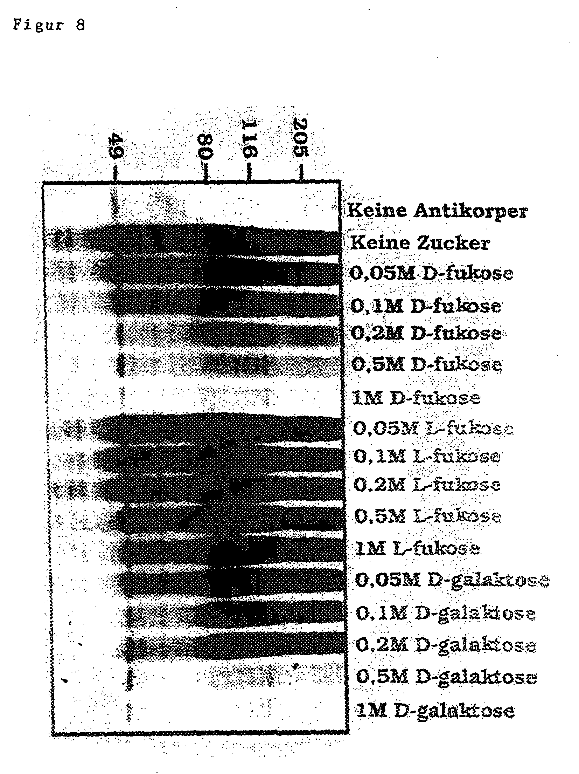

Durch Markierung von MT-450 Zellen mit radioaktiver Fucose wurde gezeigt, daß der von dem M-N#1-Antikörper erkannte, N-glykosidisch gebundene Zucker, fucosiliert ist: i). Aus Lysaten von fucosemarkierten Zellen präzipitierte der Antikörper Proteine mit ähnlichem Molekulargewicht wie aus ³⁵S-markierten Zellen (Fig. 7). ii) Behandlung der immunpräzipizierten Proteine mit N-GlykosidaseF führt zur Abspaltung der Fucose (Fig. 7). iii)Reinigung von fucosilierten Proteinen mit Ulex Europaea Lectin und anschließende Immunpräzipitation mit M-N#1-Antikörper identifizierte Proteine mit ähnlichem Molekulargewicht wie nach ³⁵S-Markierung (Fig. 7). iv) Die Bindung von Protein an M-N#1-Antikörper wird durch steigende Mengen von D-Fucose und D-Galaktose gehemmt. L-Fucose hat keinen Einfluß auf die Bindung (Fig. 8). v) Die Reaktivität des M-N#1-Antikörpers mit menschlichen Erythrozyten und vaskulärem Endothel ist abhängig vom Blutgruppenstatus. Man findet Reaktivität mit Erythrozyten von B und AB Individuen (nicht gezeigt) und mit Epithel des Pylorus und Duodenum von B Individuen, wenn sie Blutgruppenantigen sezernieren. Von A Individuen findet man Reaktion mit Epithel des Pylorus, nicht aber des Duodenums und Jejunum oder Erythrozyten, wenn die Individuen Blutgruppenantigen sezernieren (Tabelle 3). vi) Der M-N#1-Antikörper bindet schließlich in EUSA-Experimenten an menschlichen Speichel von Blutgruppe B Individuen. Diese Bindung kann durch synthetisch hergestelltem Blutgruppenzucker des Typs B2,3,4 und des Typs A2 blockiert werden, nicht aber durch Typ B1, A1,3,4 oder H. Die Blockierung der Bindung ist von 1-2 gebundener Fucose abhängig (Tabelle 5).

**Tabelle 1 Immunfluoreszenzfärbung von Mammakarzinomzellen aus Ratten**

| Zellinien | Metastasierungspotential | 5G8 | 1.1ASML | M-N#1 |
|---|---|---|---|---|
| MTLN2 | ++ | +++ | +++ | - |
| MTLN3 | +++ | ++ | ++ | - |
| MTC | + | +++ | +(+) | - |
| MTLy | +++ | ++ | ++ | ++* |
| MTPA | + | ++ | (+) | - |
| RBA | +++ | ++ | +(+) | - |
| MAT B | +++ | +++ | +++ | ++* |

### Immunfluoreszenzfärbung

Die Zellinien wurden mit den CD44 spezifischen Antikörpern 5G8 und 1. LASML (Sleeman et al., Cancer Research 56 3134-3141, 1996) und mit dem M-N#1 Antikörper durchgeführt. Die Färbungsintensität wurde subjektiv auf einer vierteiligen Skala (-, keine Färbung, +++, starke Färbung) bestimmt. Das Metastasierungspotential entspricht der Einteilung, wie sie in der Publikation Sleeman et al., 1996, Cancer Res.56, 3134-3141 vorgenommen wurde. etwa 10% der Zellen waren stark gefärbt.

**Tabelle 2 Immunhistologische Färbung von Normalgeweben**

| Gewebe | M-N#1Färbung |
|---|---|
| MT.450 Tumor | +++ |
| Gehirn | - |
| Niere | - |
| Muskel | - |
| Haut | - |
| Herz | - |
| Lunge | - |
| Leber | - |
| Lymphknoten | - |
| Thymus | - |
| Milz | - |
| Testis | - |
| Ovar | - |
| Zervix | (+) |
| E12,5Embryo | - |
| Pankreas | +++ |
| Gastrointestinal Trakt: Zunge | - |
| Gastrointestinal Trakt: Oesophagus | - |
| Gastrointestinal Trakt: Magen-Mageneingang | - |
| Gastrointestinal Trakt: Magen-Hauptmagen | +++ |
| Gastrointestinal Trakt: Magen Magenausgang | +++ |
| Gastrointestinal Trakt Duodenum | (+) |
| Gastrointestinal Trakt: Ileum | + |
| Gastrointestinal Trakt: Blindarm | - |
| Gastrointestinal Trakt: Dickdarm | + |
| Mamma Fettgewebe | - |
| Gastrointestinal Trakt Duodenum | (+) |
| Gastrointestinal Trakt: Ileum | + |
| Gastrointestinal Trakt: Blindarm | - |
| Gastrointestinal Trakt: Dickdarm | + |
| Mamma Fettgewebe | - |
| Speichekdrüre | + |
| Rückenrnark(Oberschenkelknochen) | +++ |

Paraffinschnitte wurden mit M-N#1 Antikörpern immunhistochemisch gefärbt (Material und Methoden) und subjektiv nach einer vierstufigen Skala (siehe Tabelle 1) ausgewertet.

**Tabelle 3 Immunperoxidasefärbung mit M-N#1 Antikörpern der Gastrodudenums von Individuen die für den Blutgruppenstatus ABO sezernierend oder nicht sezernierend phänotypisiert wurden**

| Phänotyp der Spender | Pylorus | | Duodenum | |
|---|---|---|---|---|
| | Oberflächenepithel | Drüse | Oberflächenepithel | Brünner's Drüse |
| A nichtsezernierend | - | - | - | - |
| B nichtsezernierend | +++^{a} | -/+++^{b} | - | ++/- |
| 0 nichtsezernierend | - | - | - | - |
| A sezernierend | +++/-^{c} | - | - . | - |
| B sezernieren | ++++ ++++/- | +++/- | ++++ | |
| 0 sezernierend - - - | | | | - |

| | | | | |
|---|---|---|---|---|
| ^{c}. starke Färbung der überwiegenden Mehrzahl von Zellen. | | | | |

**Tabelle 4 Struktur von ABH verwandten Oligosacchariden, die in Bindungsstudien verwendet wurden**

| Name | Chemische Struktur |
|---|---|
| B Disaccharid | Gala1-3Galβ1-R |
| B Trisaccharid | Gala1-3(Fucal-2)Gaβ1-R |
| B Typ 1 | Gala1-3(Fucal1-2)Galβ1-3 GcNAcβ1-R |
| B Typ 2 | Gala1-3(Fucal1-2)Galβ1-4 GlcNAcβ1-R |
| B Typ 3 | Gala1-3(Fucal1-2)Galβ1-3 GlcNAca1-R |
| B Typ 4 | Gala1-3(Fucal1-2)Galβ1-3 GlcNAcβ1-R |
| B linear | Gala1-3Galβ1-4 GlcNAcβ1-R |
| Trisaccharid | |
| A Typ 1 | GalNAca1-3(Fucal-2)Galβ1-3GlcNAc1-R |
| A Typ 2 | GalNAca1-3(Fucal-2)Galβ1-4GlcNAcβ1-R |
| A Typ 3 | GalNAca1-3(Fucal-2)Galβ1-3GalNAca1-R |
| A Typ 4 | GalNAca1-3(Fucal-2)Galβ1-3GalNAcβ1-R |
| A Le^{b} | GalNAca1-3(Fucal-2)Galβ1-3(Fucal-4)GlcNacβ1-R |
| A Le^{y} | GalNAca1-3(Fucal-2)Galβ1-4(Fucal-3)GlcNacβ1-R |
| H Typ 1 | Fucal-2Galβ1-3GlcNAcβ1-R |
| H Typ 2 | Fucal-2Galβ1-4GlcNAcβ1-R |
| H Typ 3 | Fucal-2Galβ1-3GlcNAca1-R |
| H Typ 4 | Fucal-2Galβ1-3GlcNAcβ1-R |
| R=(CH₂)₈-CO-NH-Chromosorb | |

**Tabelle 5 Bindung von M-N#1 an festgekoppelte Oligosaccharide**

| Antigen | %Adsorption |
|---|---|
| B tri | 93 |
| B Typ 1 | 0 |
| B Typ 2 | 97 |
| B Typ 3 | 97 |
| B Typ 4 | 98 |
| B Typ 5 | 68 |
| Kontrolle | 0 |

Die Bindung von M-N#1 Antikörpern an Oligosacchariden, die an einer festen Matrix fixiert sind, ist in Material und Methoden beschrieben.

### Legenden zu den Abbildungen

### Abbildung 1

Immunfluoreszenzfärbung von NM-081 und MT-450 Zellen mit dem M-N#1-Antikörper. Nur die MT-450 Zellen werden durch den Antikörper angefärbt (rote Immunfluoreszenz).

### Abbildung 2

Wirkung des M-N#1-Antikörpers auf Tumorwachstum und Überleben von tumortragenden Ratten.
Tumorwachstum in A. (Volumen des primären Tumors) und Überleben in B. von Wistar Furth Ratten, die subkutan mit 5x10⁵ MT-450 Zellen injiziert wurden und danach mit je 200 *µ*g Kontrollantikörper (IB7) oder M-N#1 zweimal pro Woche behandelt wurden, ist dargestellt.

### Abbildung 3

Immunhistochemische Färbung von Paraffinschnitten von Tumoren der MT Serie mit den M-N#1-Antikörpern

### Abbildung 4

Immunhistochemische Färbung von Paraffinschnitten von Mammageweben weiblicher BDX-Ratten nach Laktationsende mit den M-N#I-Antikörpern. Zum Zeitpunkt 0 wurden die Jungen der Ratten entfernt.

### Abbildung 5

Immunhistochemische Färbung von Paraffinschnitten von Prostatagewebe der Ratte mit dem M-N#1-Antikörper. Männliche BDX Ratten wurden zum Tag 0 kastriert und zu den angegebenen Zeiten Prostatagewebe entnommen und mit M-N#1-Antikörpern angefärbt.

### Abbildung 6

Das M-N#1-Antigen ist Teil eines N-glycosidisch gebundenen Zuckerrestes. Radioaktiv markierte Zellysate von MT-450 Zellen wurden mit M-N#1 immunpräzipitiert und die präzipitierten Proteine wurden auf einem SDS Acrylamidgel aufgetrennt.
M-N#1 präzipitierte Proteine wandern als Schmier (1. Bahn). Die Buchstaben indizieren die Behandlung der Immunpräzipitäten vor der gelelektrophoretischen Trennung: NGF: N-GlykosidaseF; OG: 0-Glykosidase; N: Neuraminidase. Re-IP.ed bedeutet, daß die enzymbehandelten Immunpräzipitate "repräzipitiert" wurden. Der rechte Teil der Abbildung zeigt das Resultat einer Tunikamyzin-Behandlung. M-N#1-T 450 Zellen wurden vor Markierung mit radioaktivem Methionin für die angegebene Zeit mit Tunikamyzin behandelt und nach Markierung immunpräzipitiert. Die Kontrolle stellt eine Präzipitation ohne Antikörper dar.

### Abbildung 7

Nachweis der Fucosylierung des M-N#1-Antigens.

MT-450 Zellen wurden mit radioaktiver Fucose markiert, und Proteine in Lysaten dieser Zellen wurden mit M-N#1 präzipitiert (Kontrolle = Präzipitation ohne Antikörper). Dargestellt ist die Auftrennung der präzipitierten Proteine auf SDS Polyacrylamidgelen. NGF (N-GlycosidaseF) indiziert immun präzipitierte Proteine, die vor der Auftrennung mit NGF behandelt wurden. Für das Experiment "U.Europaeus" (rechter Teil der Abbildung) wurden MT-450 Zellen mit ¹⁴C-Methionin markiert und die Extrakte über eine Ulex Europaeus Lektinsäule gereinigt. Gebundene Proteine wurden eluiert und ohne Antikörper (cont) oder mit M-N#1 Antikörpern präzipitiert. NGF indiziert N-GlycosidaseF Behandlung.

### Abbildung 8

Kompetition der M-N#1-Bindung mit Fucose und Galactose. Proteine in Lysaten von radioaktiv markierten (¹⁴C-Methionin) MT-450 Zellen wurden mit M-N#1-Antikörpern in Abwesenheit oder steigenden Konzentrationen von Fucose oder Galaktose immunpräzipitiert und gelelektrophoretisch aufgetrennt.

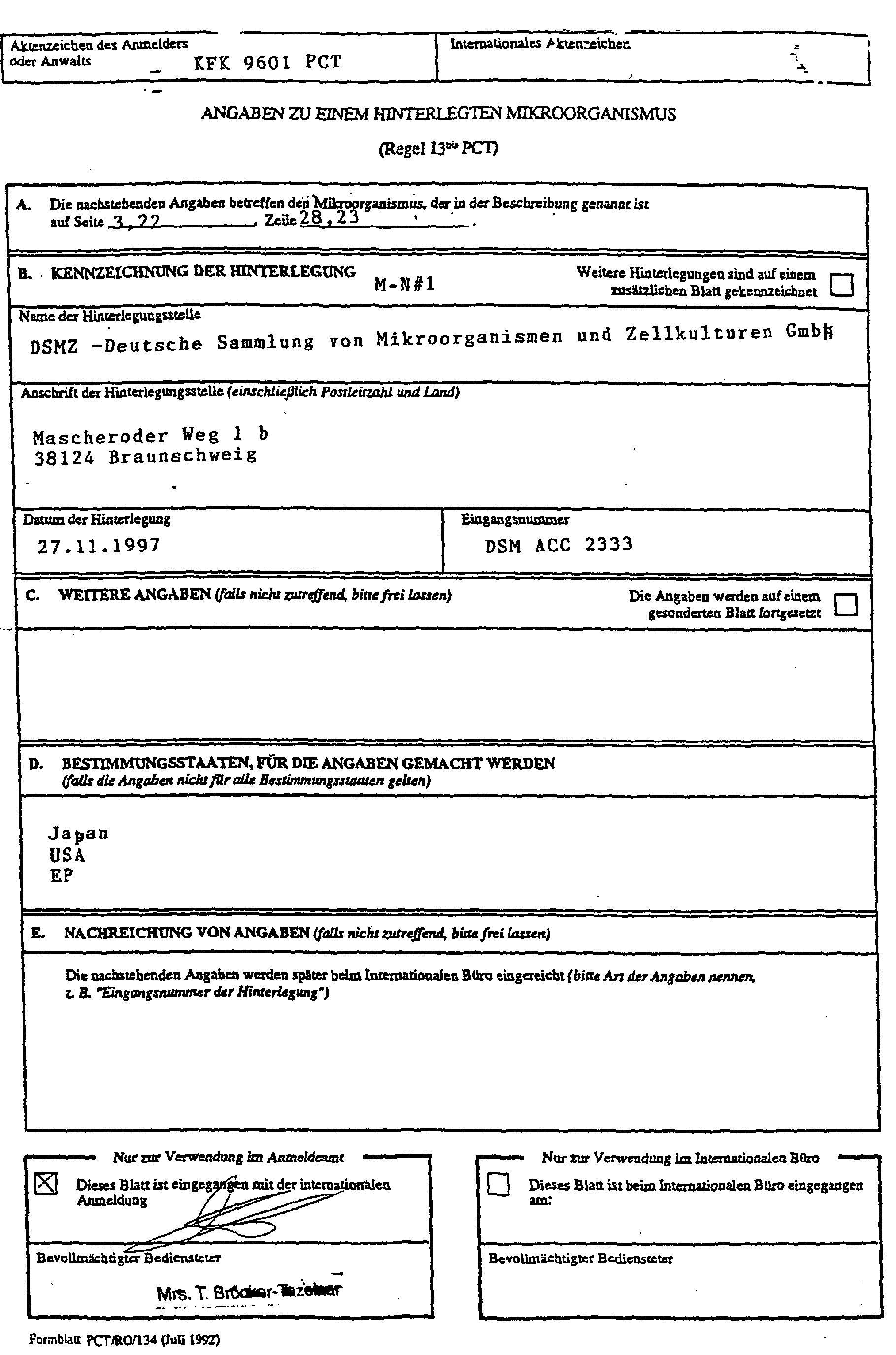
Aktenzeichen des Anmelders
oder Anwalts KFK 9601 PCT | Internationales Aktenzeichen

ANGABEN ZU EINEM HINTERLEGTEN MIKROORGANISMUS

(Regel 13bis PCT)

A. Die nachstehenden Angaben betreffen den Mikroorganismus, der in der Beschreibung genannt ist
auf Seite 3, 22 , Zeile 28, 23 .

B. KENNZEICHNUNG DER HINTERLEGUNG M-N#1 | Weitere Hinterlegungen sind auf einem zusätzlichen Blatt gekennzeichnet ☐

Name der Hinterlegungsstelle
DSMZ -Deutsche Sammlung von Mikroorganismen und Zellkulturen GmbH

Anschrift der Hinterlegungsstelle (einschließlich Postleitzahl und Land)
Mascheroder Weg 1 b
38124 Braunschweig

Datum der Hinterlegung | Eingangsnummer
27.11.1997 | DSM ACC 2333

C. WEITERE ANGABEN (falls nicht zutreffend, bitte frei lassen) | Die Angaben werden auf einem gesonderten Blatt fortgesetzt ☐

D. BESTIMMUNGSSTAATEN, FÜR DIE ANGABEN GEMACHT WERDEN
(falls die Angaben nicht für alle Bestimmungsstaaten gelten)

Japan
USA
EP

E. NACHREICHUNG VON ANGABEN (falls nicht zutreffend, bitte frei lassen)

Die nachstehenden Angaben werden später beim Internationalen Büro eingereicht (bitte Art der Angaben nennen, z. B. "Eingangsnummer der Hinterlegung")

Nur zur Verwendung im Anmeldeamt | Nur zur Verwendung im Internationalen Büro
☒ Dieses Blatt ist eingegangen mit der internationalen Anmeldung | ☐ Dieses Blatt ist beim Internationalen Büro eingegangen am:
Bevollmächtigter Bediensteter | Bevollmächtigter Bediensteter
Mrs. T. Bröcker-Tezelaar

Formblatt PCT/RO/134 (Juli 1992)

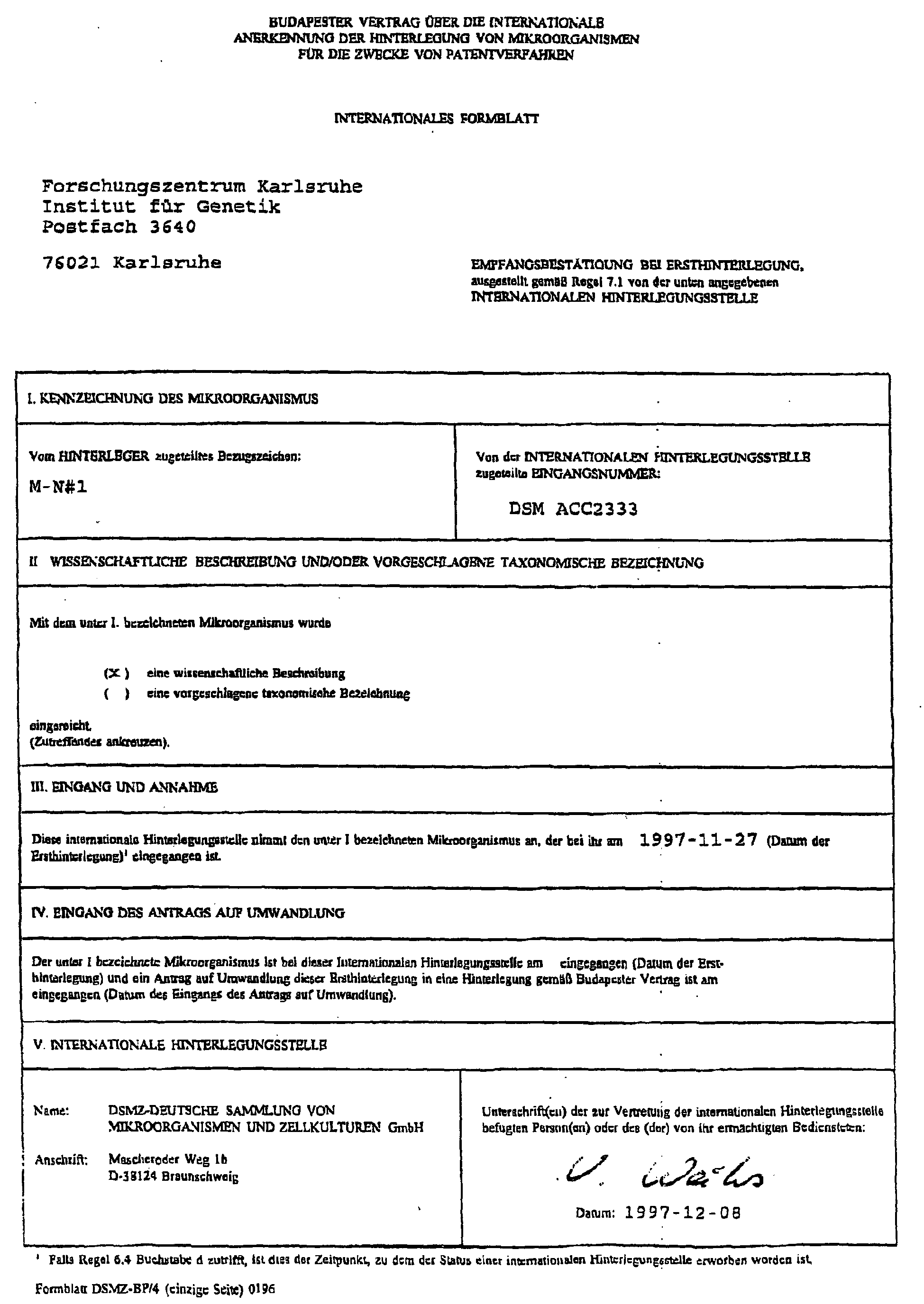
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

INTERNATIONALES FORMBLATT

Forschungszentrum Karlsruhe
Institut für Genetik
Postfach 3640

76021 Karlsruhe

EMPFANGSBESTÄTIGUNG BEI ERSTHINTERLEGUNG,
ausgestellt gemäß Regel 7.1 von der unten angegebenen
INTERNATIONALEN HINTERLEGUNGSSTELLE

I. KENNZEICHNUNG DES MIKROORGANISMUS

Vom HINTERLEGER zugeteiltes Bezugszeichen:
M-N#1 | Von der INTERNATIONALEN HINTERLEGUNGSSTELLE
zugeteilte EINGANGSNUMMER:
DSM ACC2333

II WISSENSCHAFTLICHE BESCHREIBUNG UND/ODER VORGESCHLAGENE TAXONOMISCHE BEZEICHNUNG

Mit dem unter I. bezeichneten Mikroorganismus wurde

(X) eine wissenschaftliche Beschreibung
( ) eine vorgeschlagene taxonomische Bezeichnung

eingereicht.
(Zutreffendes ankreuzen).

III. EINGANG UND ANNAHME

Diese internationale Hinterlegungsstelle nimmt den unter I bezeichneten Mikroorganismus an, der bei ihr am 1997-11-27 (Datum der
Ersthinterlegung)¹ eingegangen ist.

IV. EINGANG DES ANTRAGS AUF UMWANDLUNG

Der unter I bezeichnete Mikroorganismus ist bei dieser Internationalen Hinterlegungsstelle am eingegangen (Datum der Erst-
hinterlegung) und ein Antrag auf Umwandlung dieser Ersthinterlegung in eine Hinterlegung gemäß Budapester Vertrag ist am
eingegangen (Datum des Eingangs des Antrags auf Umwandlung).

V. INTERNATIONALE HINTERLEGUNGSSTELLE

Name: DSMZ-DEUTSCHE SAMMLUNG VON
MIKROORGANISMEN UND ZELLKULTUREN GmbH

Anschrift: Mascheroder Weg 1b
D-38124 Braunschweig | Unterschrift(en) der zur Vertretung der internationalen Hinterlegungsstelle
befugten Person(en) oder des (der) von ihr ermächtigten Bediensteten:


Datum: 1997-12-08

¹ Falls Regel 6.4 Buchstabe d zutrifft, ist dies der Zeitpunkt, zu dem der Status einer internationalen Hinterlegungsstelle erworben worden ist.

Formblatt DSMZ-BP/4 (einzige Seite) 0196

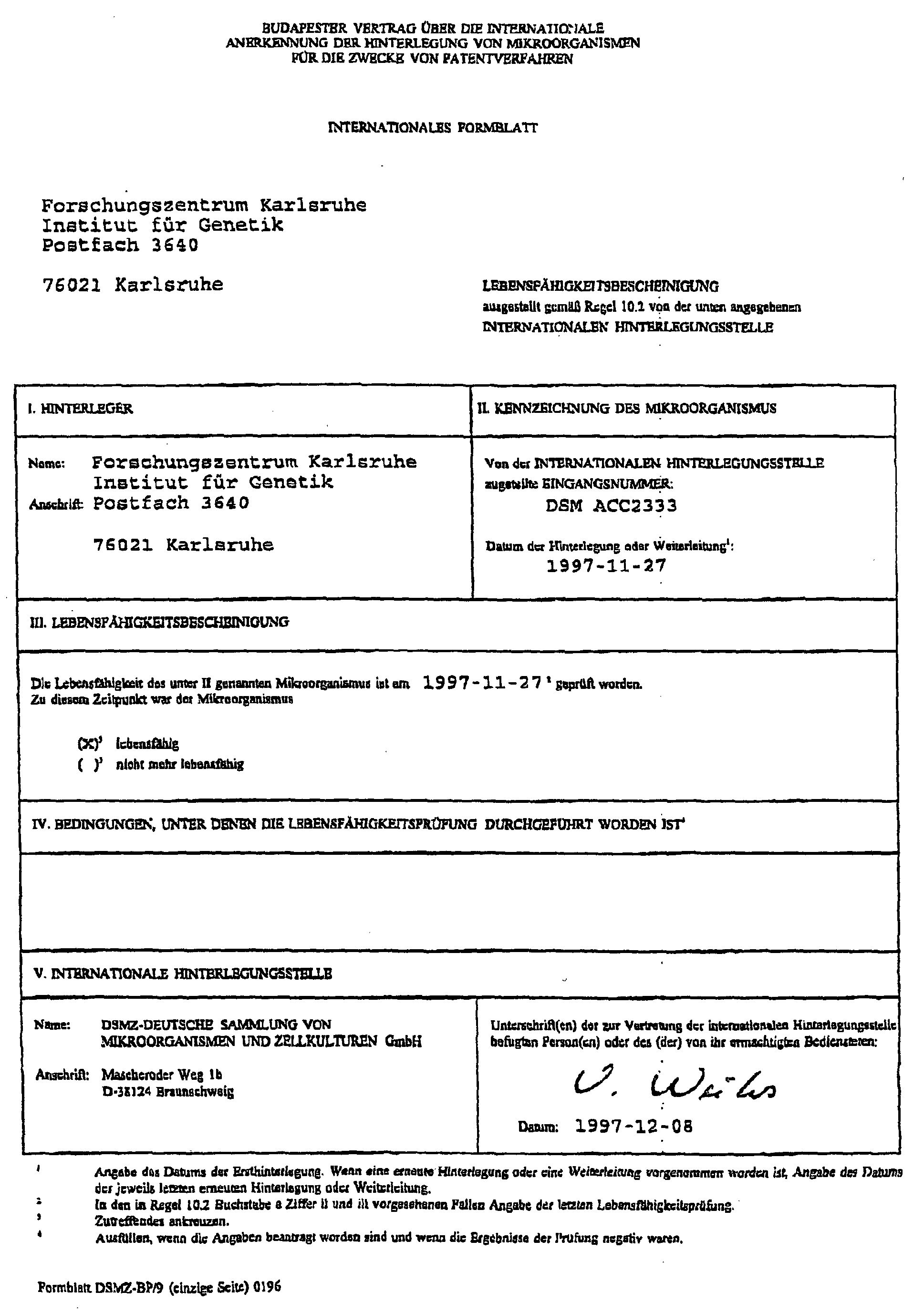
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

INTERNATIONALES FORMBLATT

Forschungszentrum Karlsruhe
Institut für Genetik
Postfach 3640

76021 Karlsruhe

LEBENSFÄHIGKEITSBESCHEINIGUNG
ausgestellt gemäß Regel 10.2 von der unten angegebenen
INTERNATIONALEN HINTERLEGUNGSSTELLE

I. HINTERLEGER | II. KENNZEICHNUNG DES MIKROORGANISMUS
Name: Forschungszentrum Karlsruhe
Institut für Genetik
Anschrift: Postfach 3640

76021 Karlsruhe | Von der INTERNATIONALEN HINTERLEGUNGSSTELLE
zugeteilte EINGANGSNUMMER:
DSM ACC2333

Datum der Hinterlegung oder Weiterleitung¹:
1997-11-27

III. LEBENSFÄHIGKEITSBESCHEINIGUNG

Die Lebensfähigkeit des unter II genannten Mikroorganismus ist am 1997-11-27 ² geprüft worden.
Zu diesem Zeitpunkt war der Mikroorganismus

(X)³ lebensfähig
( )³ nicht mehr lebensfähig

IV. BEDINGUNGEN, UNTER DENEN DIE LEBENSFÄHIGKEITSPRÜFUNG DURCHGEFÜHRT WORDEN IST⁴

V. INTERNATIONALE HINTERLEGUNGSSTELLE

Name: DSMZ-DEUTSCHE SAMMLUNG VON
MIKROORGANISMEN UND ZELLKULTUREN GmbH

Anschrift: Mascheroder Weg 1b
D-38124 Braunschweig | Unterschrift(en) der zur Vertretung der internationalen Hinterlegungsstelle
befugten Person(en) oder des (der) von ihr ermächtigten Bediensteten:


Datum: 1997-12-08

¹ Angabe des Datums der Ersthinterlegung. Wenn eine erneute Hinterlegung oder eine Weiterleitung vorgenommen worden ist, Angabe des Datums der jeweils letzten erneuten Hinterlegung oder Weiterleitung.
² In den in Regel 10.2 Buchstabe a Ziffer ii und iii vorgesehenen Fällen Angabe der letzten Lebensfähigkeitsprüfung.
³ Zutreffendes ankreuzen.
⁴ Ausfüllen, wenn die Angaben beantragt worden sind und wenn die Ergebnisse der Prüfung negativ waren.

Formblatt DSMZ-BP/9 (einzige Seite) 0196

## Patentansprüche

1. Mittel enthaltend Antikörper, die mit Glycoproteiden menschlicher oder tierischer Herkunft, enthaltend N-glycosidisch gebundene Saccharide des blutgruppenspezifischen Antigen-Typs B2, 3, 4 oder A2 reagieren, zur Verwendung bei der Identifizierung und Unterdrückung des Wachstums von Tumorzellen.

2. Mittel nach Anspruch 1 enthaltend einen monoklonalen Antikörper M-N#1, der aus der Zellkultur mit der Hinterlegungsnummer DSM ACC 2333 erhältlich ist.

3. Verwendung des Mittels nach einem der Ansprüche 1 bis 2 zur Herstellung einer enteral, parenteral, systemisch, lokal und/oder topisch zu verabreichenden Apptikationsform.

4. Verwendung des Mittels nach einem der Ansprüche 1 bis 2 zur Herstellung einer zur Injektion, Infusion oder Perfusion geeigneten Darreichungsform.

5. Verwendung eines Mittels gemäß einem der Ansprüche 1 bis 2 zur Identifizierung von Tumorzellen in vitro.

## Claims

1. Composition comprising antibodies which react with glycoproteins of human or animal origin, comprising N-glycosidically bonded saccharides of the blood group-specific antigen of type B2, 3, 4 or A2, to be used for identifying and suppressing the growth of tumour cells.

2. Composition according to Claim 1, comprising a monoclonal M-N#1 antibody which is obtainable from the cell culture with the deposit number DSM ACC 2333.

3. Use of the composition according to any of Claims 1 to 2, for the production of an administration form for enteral, parenteral, systemic, local and/or topical administration.

4. Use of the composition according to any of Claims 1 to 2, for the production of a dosage form suitable for injection, infusion or perfusion.

5. Use of a composition according to any of Claims 1 to 2 for identifying tumour cells in vitro.

## Revendications

1. Agent contenant des anticorps qui réagissent avec des glycoprotéides d'origine humaine ou animale, contenant des saccharides liés par une liaison N-glycosidique du type d'antigène B2, 3, 4 ou A2 spécifique des groupes sanguins, destiné à être utilisé lors de l'identification et la suppression de la croissance de cellules tumorales.

2. Agent selon la revendication 1, contenant un anticorps monoclonal M-N#1 pouvant être obtenu à partir de la culture cellulaire présentant le numéro de dépôt DSM ACC 2333.

3. Utilisation de l'agent selon la revendication 1 ou 2 pour la préparation d'une forme d'administration par voie entérale, parentérale, systémique, locale et/ou topique.

4. Utilisation de l'agent selon la revendication 1 ou 2 pour la préparation d'une forme d'administration appropriée à une injection, une infusion ou une perfusion.

5. Utilisation d'un agent selon la revendication 1 ou 2 pour l'identification de cellules tumorales in vitro.
